# EUROPEAN PATENT APPLICATION

(11) **EP 3 040 021 A1**
(43) Date of publication of application: **06.07.2016**
(21) Application number: 14840090.6
(22) Date of filing: 09.06.2014
(51) Int. Cl.: A61B 5/107, G06T 1/00, G06T 7/60

(54) **ORGAN IMAGING APPARATUS**

(30) Priority: 28.08.2013 JP 2013176483
(71) Applicant: Konica Minolta, Inc., Tokyo 100-7015 (JP)
(72) Inventor: MATSUDA, Shinya, Tokyo 100-7015 (JP); KATAGIRI, Tetsuya, Tokyo 100-7015 (JP)
(74) Representative: Henkel, Breuer & Partner
(86) International application number: PCT/JP2014/065169
(87) International publication number: WO 2015/029537

(57) **Abstract**

The organ imaging apparatus (1) is provided with an image pickup unit (3), a detection unit, and an illumination unit (2). The image pickup unit (3) acquires an image by photographing an imaging object comprising an organ of the living body. The detection unit detects a feature of the imaging object on the basis of the image acquired by the image pickup unit (3). The illumination unit (2) illuminates the imaging object at an angle that exceeds 45 degrees and is not more than 90 degrees with respect to the imaging optical axis (X) of the image pickup unit (3) that passes through the imaging object. The image pickup unit (3) photographs the shadows formed by the imaging object under illumination by the illumination unit (2). The detection unit detects the topography of the imaging object and the border of the organ contained in said imaging object on the basis of the shadows.

## Description

### Technical Field

The present invention relates to an organ imaging device for imaging an organ of a living body to detect features of the organ.

### Background Art

In Oriental medicine, a technique of diagnosing health condition and disease condition by examining the condition of the human tongue (tongue diagnosis) is known. Also in Western medicine, a technique of diagnosing diseases in the circulatory or digestive organs by examining the color and the bag under the human eye (at the lower eyelid) is known. Poor metabolism or circulation of blood or other body fluid can cause an excess or deficit of blood or other body fluid in the tongue or in the skin under the eye, producing surface irregularities (dents and bumps). Examining these surface irregularities helps make a diagnosis.

Such diagnoses are made by specialized physicians, who, however, rely on experience and intuition. Thus, diagnoses tend to vary from one practitioner to another and be less than objective. Moreover, obscure memories of past condition hamper an objective grasp of changes in condition.

As a solution, there have been proposed systems in which a subject is imaged with a camera and the subject's health condition is diagnosed based on the taken image. For example, according to Patent Document 1 identified below, from an image of the tongue imaged with a digital camera, an area of interest, such as a tongue-tip or tongue-middle area, is extracted, and this is compared with a standard image stored in a database to make a diagnosis of health condition. According to Patent Document 2 identified below, from the image data of the tongue imaged, condition parameters based on the shape, surface color, etc. of the tongue are extracted, and these are compared with tongue diagnosis data (defining correlation between previously stored condition parameters and those obtained through tongue diagnosis) to derive a tongue diagnosis result.

According to Patent Document 3 identified below, an image of the tongue is taken with a digital camera equipped with a flash light source, so that image data is acquired with the color and lightness of a target region emphasized. According to Patent Document 4 identified below, the tongue is imaged alternately with a luster light source off and on to acquire image data containing only the hues of the tongue surface, excluding its luster, and image data containing both the hues and luster of the tongue surface; the difference calculated by subtracting the former image data from the latter image data is then calculated to acquire image data containing only the luster.

### List of Citations

### Patent Literature

Patent Document 1: Japanese Patent Application Publication No. 2004-209245 (see paragraphs [0013], [0014], etc.)
Patent Document 2: Japanese Patent Application Publication No. 2009-28058 (see claim 1, paragraph [0011], etc.)
Patent Document 3: Japanese Patent Application Publication No. 2006-149679 (see claim 7, paragraph [0028], etc.)
Patent Document 4: Japanese Patent Application Publication No. 2011-239926 (see claim 1, paragraphs [0021], [0024], [0025], Fig. 2, etc.)

### Summary of the Invention

### Technical Problem

Inconveniently, according to Patent Document 4, the camera is arranged straight in front of the imaging object, and the imaging object (tongue) is illuminated at a small angle (e.g., 10 degrees or more but 45 degrees or less) relative to the lens optical axis of the camera; thus, the taken image shows no shadings. This prevents accurate detection of the size of surface irregularities. For example, teeth marks on the tongue (undulating marks of teeth at the edge of the tongue, formed by teeth making contact with the tongue), or a bag under the eye, can have surface irregularities as large as about 0.5 mm. An image taken under illumination at a small angle, however, does not show shadings produced by surface irregularities, and this makes it difficult to accurately detect small surface irregularities. It is thus difficult to accurately detect teeth marks or eye bags.

When the tongue is imaged, since its hues are similar to the hues of the background, such as the face skin and the lips, from the taken image, it is difficult to accurately detect the boundary between the tongue and its surroundings; this makes it difficult to extract the area (contour) of the tongue. As a result, it is difficult to make a diagnosis based on the shape of the tongue.

Based on the foregoing, a technique of imaging an imaging object at a small angle of 45 degrees or less is not considered to contribute to improved diagnosis accuracy.

In Patent Documents 1 to 3, no particulars are given about the angle at which the imaging object is illuminated. In Patent Document 1 in particular, although the imaging object is imaged in an environment of a predetermined illuminance, no mention is made of illuminating the imaging object (e.g., the tongue) from a particular direction. Also in Patent Document 2, although it is mentioned that the color of an image of the imaging object (e.g., the tongue) varies with the environmental conditions, such as illumination, under which the image is taken, no mention is made of imaging the imaging object under illumination from a particular direction.

Devised against the background discussed above, the present invention aims to provide an organ imaging device with which it is possible to accurately detect surface irregularities on an imaging object and the boundary of an organ included in the imaging object, and thereby to improve the accuracy of a diagnosis.

### Means for Solving the Problem

According to one aspect of the present invention, an organ imaging device includes an imager which images an imaging object including an organ of a living body to acquire an image, and a detector which detects a feature of the imaging object based on the image acquired by the imager. Here, the organ imaging device further includes an illuminator which illuminates the imaging object at an angle larger than 45 degrees but equal to or smaller than 90 degrees relative to the imaging optical axis of the imager running through the imaging object. The imager images shadings formed by the imaging object under illumination by the illuminator. The detector detects a surface irregularity on the imaging object and the boundary of an organ included in the imaging object based on the shadings.

### Advantageous Effects of the Invention

With the above configuration, while an imaging object is illuminated at an angle larger than 45 degrees but equal to or smaller than 90 degrees relative to the imaging optical axis, the shadings formed by the imaging object are imaged, and based on the shadings, surface irregularities on the imaging object and the boundary of an organ included in the imaging object are detected. In this way, even when surface irregularities on the imaging object are small, they can be detected accurately, and the boundary of an organ included in the imaging object can be detected accurately. By making a diagnosis based on the results of the detection, it is possible to improve the accuracy of the diagnosis.

### Brief Description of Drawings

[Fig. 1] is a perspective view showing the exterior appearance of an organ imaging device according to one embodiment of the present invention;
[Fig. 2] is a block diagram showing an outline of the configuration of the organ imaging device;
[Fig. 3] is an explanatory diagram illustrating illumination angles with respect to an imaging object;
[Fig. 4] is an explanatory diagram schematically showing images taken at illumination angles of 15 degrees and 60 degrees, respectively, with a tongue as an imaging object;
[Fig. 5] is an explanatory diagram schematically showing images taken at illumination angles of 15 degrees and 60 degrees, respectively, with an eye and an area under it as an imaging object;
[Fig. 6] is an explanatory diagram schematically showing images taken at illumination angles of 15 degrees and 60 degrees, respectively, along with the areas of the tongue extracted from those taken images;
[Fig. 7] is an explanatory diagram showing a chart of a distribution of luminance values of pixels on line E-E' in the image taken at an illumination angle of 60 degrees in Fig. 6, along with a chart of luminance value differences between consecutive pixels in direction E-E';
[Fig. 8] is an explanatory diagram schematically showing taken images with and without teeth marks respectively, along with the areas of the tongue extracted from those taken images;
[Fig. 9] is an explanatory diagram schematically showing taken images with and without a bag under an eye respectively, along with the luminance distributions of those taken image;
[Fig. 10] is a flow chart showing the flow of operation in the organ imaging device;
[Fig. 11] is an explanatory diagram schematically showing another configuration of the organ imaging device;
[Fig. 12] is an explanatory diagram schematically showing yet another configuration of the organ imaging device; and
[Fig. 13] is an explanatory diagram schematically showing an outline of the structure of a color filter provided in the imager of the organ imaging device shown in Fig. 12.

### Description of Embodiments

An embodiment of the present invention will be described below with reference to the accompanying drawings.

### [Overall Configuration of an Organ Imaging Device]

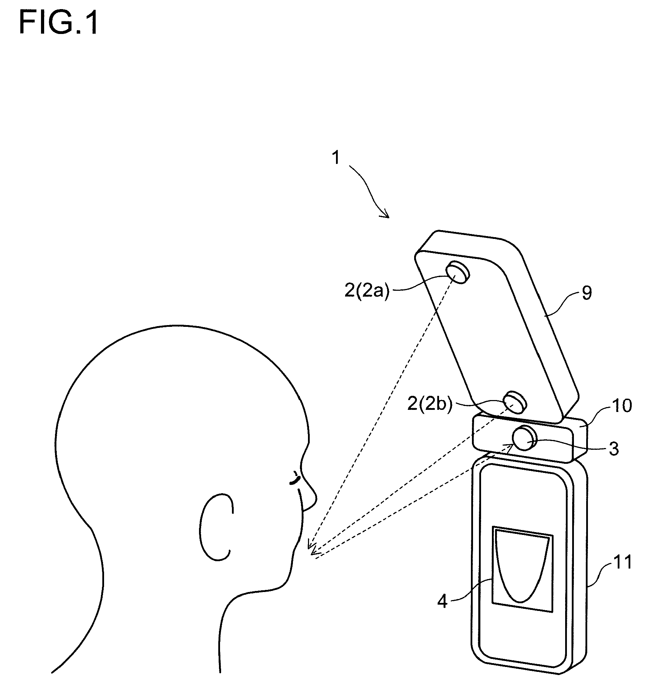

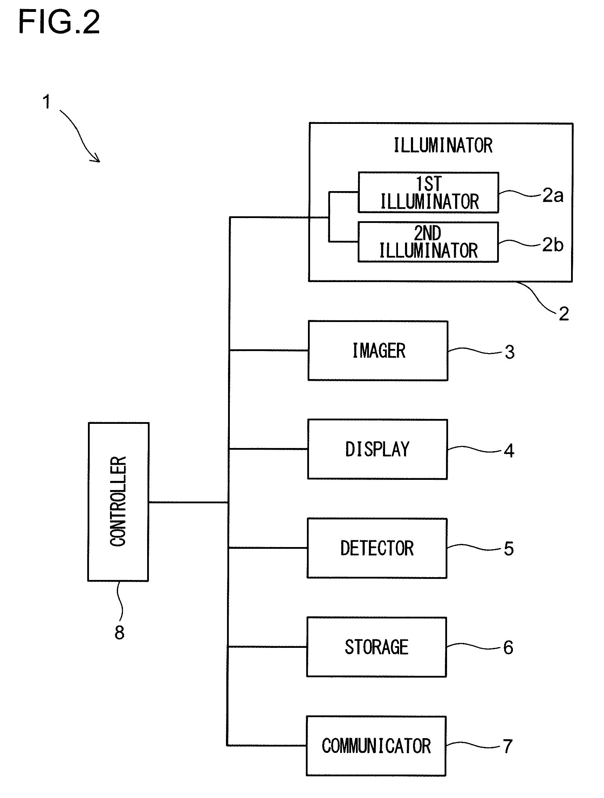

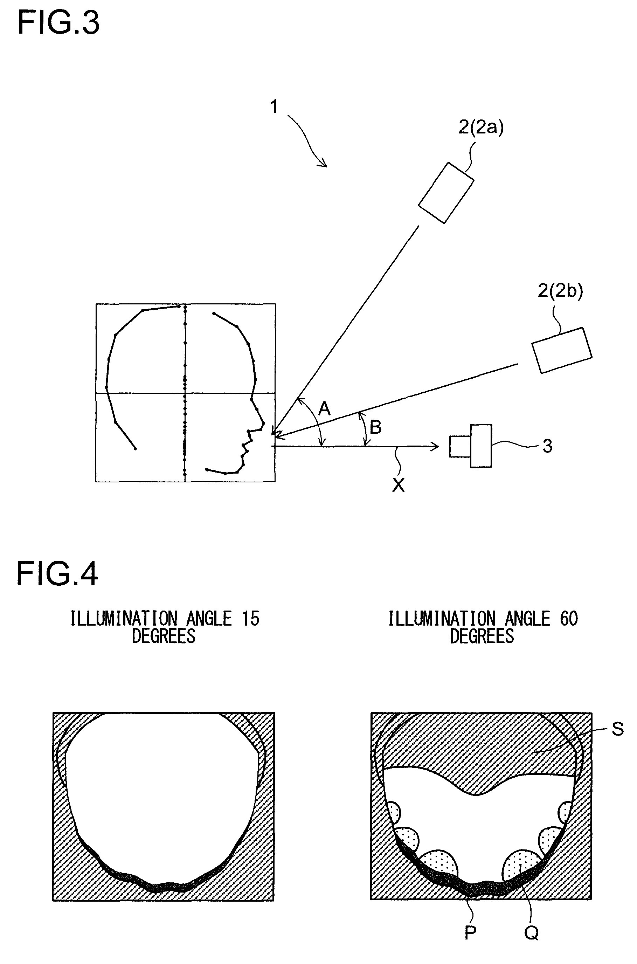

Fig. 1 is a perspective view showing the exterior appearance of an organ imaging device 1 according to one embodiment of the present invention. Fig. 2 is a block diagram showing an outline of the configuration of the organ imaging device 1. Fig. 3 is an explanatory diagram showing illumination angles with respect to an imaging object. In this embodiment, objects of imaging by the organ imaging device 1 include organs of a living body (a tongue, an eye, etc.) and parts surrounding them (e.g., an area around a tongue, an area around (in particular, under) an eye, etc.).

The organ imaging device 1 includes an illuminator 2, an imager 3, a display 4, a detector 5, a storage 6, a communicator 7, and a controller 8. The illuminator 2, the imager 3, and the display 4 are provided in a housing 9, in a support 10, and in a housing 11 respectively. The housing 9 and the support 10 are coupled together such that these are rotatable relative to each other both in the up/down direction and in the horizontal direction, and so are the support 10 and the housing 11. The detector 5, the storage 6, the communicator 7, and the controller 8 are provided, for example, in the housing 11.

The illuminator 2 illuminates an imaging object. The illuminator 2 is composed of a first illuminator 2a which illuminates the imaging object from a relatively upper direction, and a second illuminator 2b which illuminates the imaging object from a relatively lower direction. The first illuminator 2a illuminates the imaging object at an angle A larger than 45 degrees but equal to or smaller than 90 degrees relative to the imaging optical axis X of the imager 3 which runs through the imaging object. On the other hand, the second illuminator 2b illuminates the imaging object at an angle B smaller than 45 degrees relative to the imaging optical axis X. The imaging optical axis X denotes the optical axis of an imaging lens included in the imager 3. Although in this embodiment the illuminator 2 includes the first and second illuminators 2a and 2b, it can instead include the first illuminator 2a alone (the illuminator 2 does not have to include the second illuminator 2b).

The illuminator 2 (first and second illuminators 2a and 2b) includes a light source, which is implemented with a light source that emits light of a daylight color, such as a xenon lamp, for improved color reproduction. The brightness of the light source, though it depends on the sensitivity of the imager 3 and the distance to the imaging object, is, for example, such that the illuminance at the imaging object is 1 000 to 10 000 1x.

The first and second illuminators 2a and 2b each further include, in addition to the light source, a lighting circuit and a dimmer circuit, and are controlled by instructions from the controller 8 so as to be turned on and off and be dimmed.

With the imaging object illuminated by the illuminator 2, the imager 3 images the imaging object to acquire an image, and also images the shadows and shades (collectively referred to as shadings, which will be discussed in detail later) formed by the imaging object. The imager 3 includes an imaging lens and an area sensor (image sensor). The aperture of the imaging lens, the shutter speed, and the focal length are so set that the entire area of the imaging object is in focus. For example, the f-number is set at 16, the shutter speed is set at 1/120 seconds, and the focal length is set at 20 mm.

The area sensor is implemented with an image sensor such as a CCD (charge-coupled device) or a CMOS (complementary metal-oxide-semiconductor), and is set at such a sensitivity, a resolution, etc. that it can satisfactorily detect the color and shape of the imaging object. For example, it is set at a sensitivity of 60 db and a resolution of 10 million pixels.

The imaging by the imager 3 is controlled by the controller 8. The imager 3 further includes, in addition to the imaging lens and the area sensor, a focusing mechanism, an aperture mechanism, a driving circuit, an A/D conversion circuit, etc., though none of these is illustrated. In response to instructions from the controller 8, the imager 3 controls focusing, the aperture, A/D conversion, etc. The imager 3 acquires, as the data of a taken image, for example, data containing eight-bit values, each between 0 and 255, for each of red (R), green (G), and blue (B).

The display 4 includes a liquid crystal panel, a backlight, a lighting circuit, and a control circuit, through none of these is illustrated. In response to instructions from the controller 8, the display 4 displays the image taken by the imager 3. It can also display information (e.g., results of diagnosis conducted based on a taken image transmitted to a remote medical institution) acquired from the outside via the communicator 7, which will be described later.

The detector 5 includes an unillustrated data processor, and detects features of the imaging object based on the image (data) acquired by the imager 3. For example, the detector 5 can convert RGB image data from the imager 3 into YCC data comprising a luminance signal Y and color-difference signals Cr and Cb and thereby acquire luminance information on the imaging object, and then, based on the acquired luminance information, detect irregularities (dents and bumps) on the surface of the imaging object, the contour (boundary) of an organ, etc. Examples of how a feature of the imaging object is detected will be described later.

The storage 6 is memory for storage of data of images acquired by the imager 3, information resulting from detection by the detector 5, information received from the outside, etc. The communicator 7 is an interface via which to transmit information resulting from detection by the detector 5 to the outside over a communication line (which can be wired or wireless) and to receive information from the outside. The controller 8 controls the operation of different parts of the organ imaging device 1, and includes, for example, a CPU (central processing unit) and memory for storage of programs for controlling the different parts.

### [Example of Arrangement of the Illuminator and the Imager]

Next, a description will be given of an example of arrangement of the illuminator 2 and the imager 3. As shown in Fig. 3, the imager 3 is arranged straight in front of a person's tongue, eyes, and face as an imaging object.

The first illuminator 2a is arranged such that the angle A at which it illuminates the imaging object (i.e., the angle of the illumination light) is about 60 degrees relative to the imaging optical axis X of the imager 3. The arrangement position of the first illuminator 2a can be adjusted by an unillustrated adjustment mechanism, which permits the illumination angle of the first illuminator 2a to be adjusted in the range larger than 45 degrees but equal to or smaller than 90 degrees.

As the illumination angle of the first illuminator 2a increases, the shadings become larger, whereas, due to the presence of the upper eyelids and the shadows of the upper lip, the area that can be imaged becomes smaller. On the other hand, as the illumination angle of the first illuminator 2a decreases, the shadings become smaller, while the area that can be imaged becomes larger.

The second illuminator 2b is arranged such that its illumination angle (angle B) is about 15 degrees relative to the imaging optical axis X of the imager 3. The arrangement position of the second illuminator 2b too can be adjusted by an unillustrated adjustment mechanism, which permits the illumination angle of the second illuminator 2b to be adjusted in the range larger than 0 degrees but equal to or smaller than 45 degrees.

As the illumination angle of the second illuminator 2b increases, due to the presence of the upper eyelids and the shadows of the upper lip, the area that can be imaged becomes smaller. On the other hand, as the illumination angle of the second illuminator 2b decreases, color clipping due to regular reflection becomes severer.

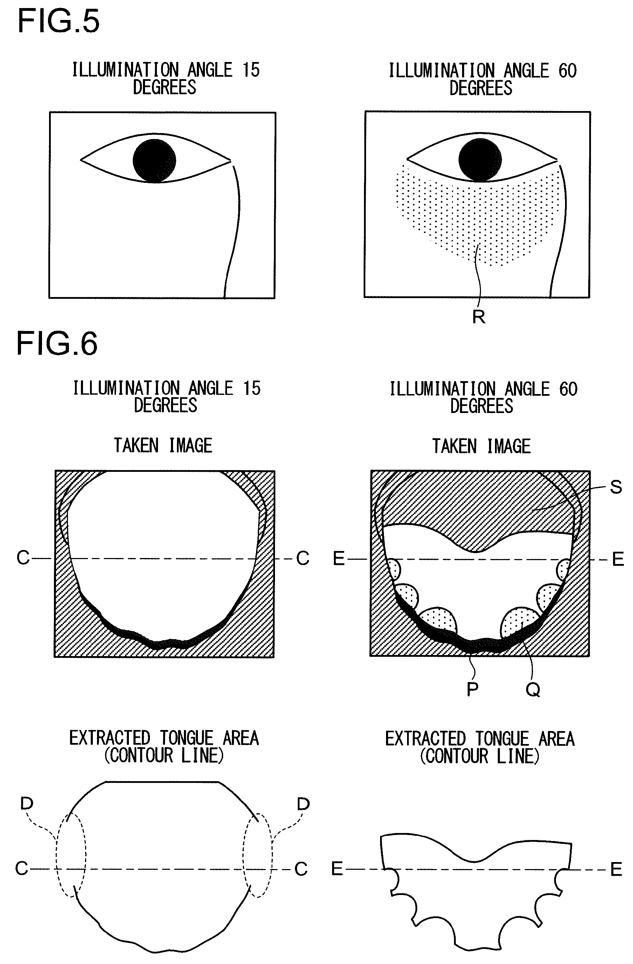

Figs. 4 and 5 are explanatory diagrams schematically showing, respectively, an image taken under the illumination by the second illuminator 2b (at an illumination angle of 15 degrees) and an image taken under the illumination by the first illuminator 2a (at an illumination angle of 60 degrees), Fig. 4 showing a case where the imaging object is a tongue, Fig. 5 showing a case where the imaging object is an eye and an area under it.

At an illumination angle of 15 degrees, whereas the entire tongue can be imaged, the shadings that represent the contour of, and teeth marks on, the tongue are small. Similarly, with respect to a bag under (in a lower part of, in an area under) the eye, the shadings representing it are small (hardly any shadings can be observed).

By contrast, at an illumination angle of 60 degrees, whereas an upper part of the tongue is not imaged due to the shadow S of the upper lip, the shadings P that represent the contour and the shadings Q that represent teeth marks are large. Similarly, with respect to a bag under the eye, the shadings R representing it are large.

From the foregoing, it is understood that, by taking an image with an imaging object illuminated at an illumination angle of 60 degrees, it is possible to accurately extract the contour (area) of a tongue and to accurately detect teeth marks on a tongue or a bag under an eye. Specific examples of how this is done will now be described.

### [Extraction of an Area]

Fig. 6 is an explanatory diagram schematically showing images obtained by imaging a tongue as an imaging object at illumination angles of 15 degrees and 60 degrees respectively and the areas (contour lines) of the tongue extracted from those images respectively. In the image taken at an illumination angle of 15 degrees, the left and right ends of a central part (along line C-C') of the tongue makes contact with the lips, and in those parts of the tongue, the shadings are small. In this case, the contour line of the tongue is discontinuous where the shadings are small (see parts D), and this makes it difficult to extract the tongue's area. Since the tongue and the lips are similar in color, even luminance information is supplemented with color information, it is still difficult to accurately extract the area of the tongue. By contrast, in the image taken at an illumination angle of 60 degrees, shadings (shadings P and Q, and a shadow S) appear around the entire circumference of the tongue, and this makes it possible to accurately extract the tongue's area.

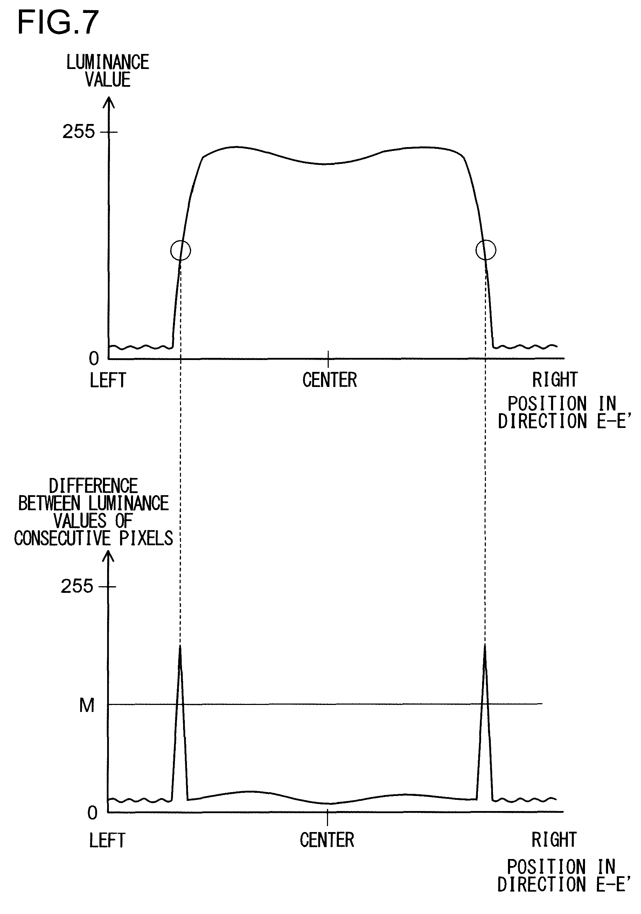

Extraction of the area of an organ (in the example above, a tongue) from a taken image can be achieved based on luminance information obtained from the taken image. Fig. 7 shows a chart of a distribution of the luminance values of pixels on line E-E' in the image taken at an illumination angle of 60 degrees in Fig. 6, along with a chart of luminance value differences (hereinafter also referred to simply as differences) between consecutive pixels in direction E-E'. Luminance values are, for example, eight-bit values each between 0 (black) and 255 (white).

In the image taken at an illumination angle of 60 degrees, luminance values vary greatly between where shadings are present and where shadings are absent; accordingly, differences calculated between every two consecutive pixels in direction E-E' are large near the boundary of the tongue. Thus, by extracting those consecutive pixels which exhibit differences exceeding a threshold value M, and repeating the process sequentially in the direction perpendicular to direction E-E' in the taken image, it is possible to extract the area of the tongue.

On the other hand, in the image taken at an illumination angle of 15 degrees, differences between consecutive pixels in direction C-C' are small in parts D in Fig. 6, and this makes it difficult to extract consecutive pixels located near the contour through comparison of differences with the threshold value, with the result that the contour line of the tongue is discontinuous.

### [Detecting Teeth marks]

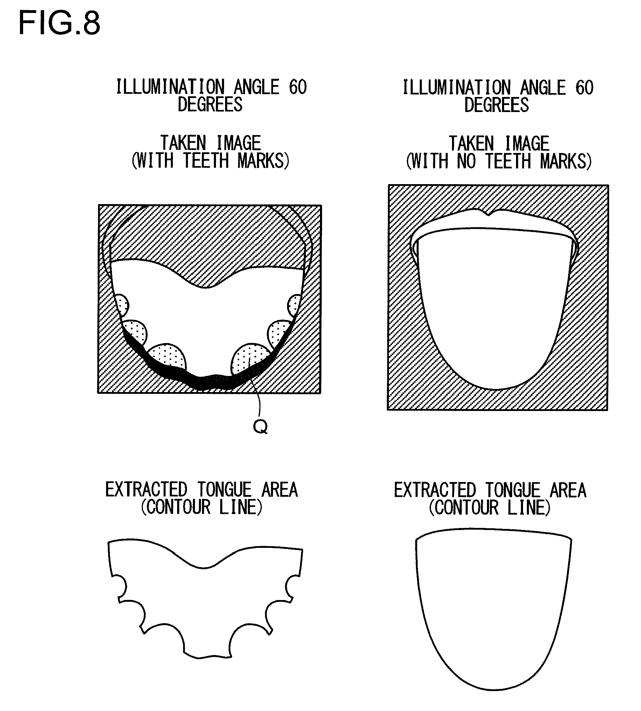

Fig. 8 is an explanatory diagram schematically showing taken images with and without teeth marks respectively, along with the areas (contour lines) of the tongue extracted from those taken images respectively. Both images are taken at an illumination angle of 60 degrees. The tongue's area is extracted in a similar manner as in Fig. 6.

When a tongue has teeth marks, the taken image shows shadings Q that reflect surface irregularities due to the teeth marks. By exploiting the shadings Q to extract a contour line based on luminance information as described previously, it is possible to detect teeth marks (surface irregularities). That is, as shown in Fig. 8, when the tongue has teeth marks, it has more irregularities along the contour line; when the tongue has no teeth marks, it has less irregularities along the contour line. Thus, based on the shape of the contour line, it is possible to recognize whether or not there are teeth marks. Moreover, by quantifying (digitizing) the length and smoothness of the contour line, it is possible to recognize the size of teeth marks.

### [Detecting a Bag Under an Eye]

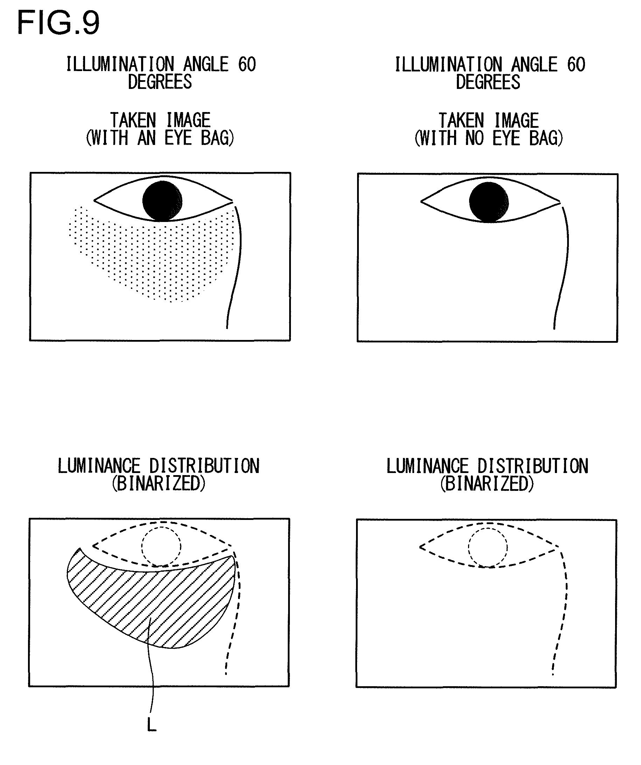

Fig. 9 is an explanatory diagram schematically showing taken images with and without a bag under an eye respectively, along with the luminance distributions in those taken images respectively. Both images are taken at an illumination angle of 60 degrees. The luminance distribution is binarized results of comparison of the luminance information of the pixels of each taken image with a threshold value.

When there is a bag under an eye, shadings due to it (surface irregularities) appear, producing a low-luminance area L under the eye. By contrast, when there is no bag under an eye, no shadings due to a bag appear, producing no low-luminance area L under the eye. Thus, by quantifying the size of the low-luminance area, it is possible to recognize whether or not there is (the size of) a bag under the eye.

Although, here, whether there is a bag or not is detected through binarization of luminance information, it is also possible to extract the contour line of a bag in a similar manner as in Fig. 6 and detect, based on the shape of the contour, whether there is a bag or not.

### [Control Flow]

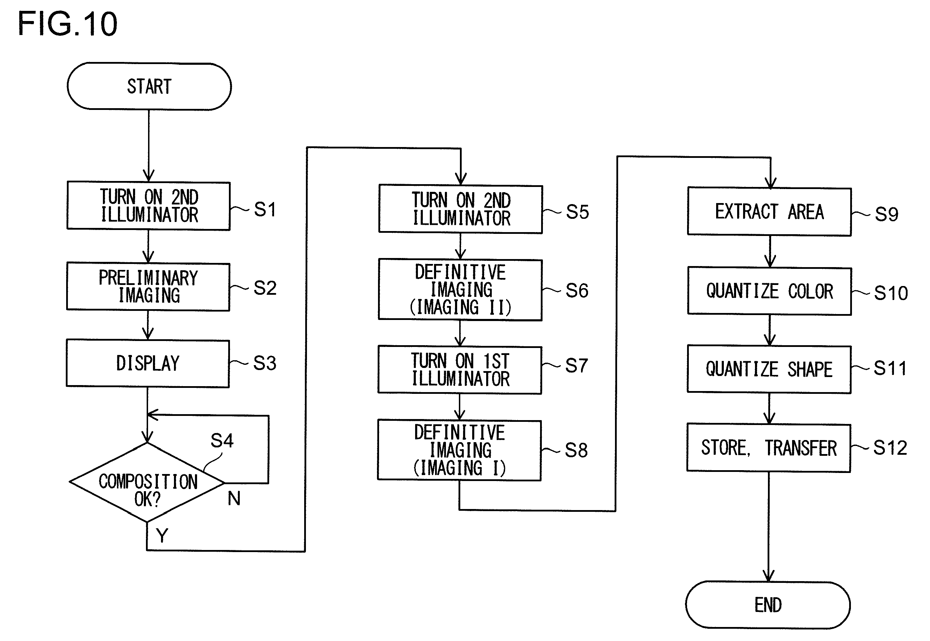

Fig. 10 is a flow chart showing the flow of operation in the organ imaging device 1 according to the embodiment under discussion. On receiving an instruction to take an image from an unillustrated input handler, the organ imaging device 1 operates as follows. The controller 8 turns on the second illuminator 2b of the illuminator 2 (S1), and the imager 3 images an imaging object (S2) on a preliminary basis. Then, the taken image is displayed on the display 4 (S3), and a user is prompted to confirm the picture composition in terms of the arrangement and angle of the imaging object, the state of a tongue stuck out, etc. (S4).

When, at S4, the picture composition is determined (accepted), the controller 8 turns on the second illuminator 2b once again (S5), and the imager 3 images the imaging object on a definitive basis (imaging II) (S6). Subsequently, the controller 8 turns on the first illuminator 2a (S7), and the imager 3 images the imaging object on a definitive basis (imaging I) (S8).

On completion of definitive imaging (imaging I), the detector 5 extracts, from the image taken in imaging I, an area of the imaging object (e.g., a tongue, or a bag under an eye) based on luminance information (S9). Based on the data of the taken image (e.g., RGB image data) and by well-known techniques, the detector 5 also detects the color and luster inside the area and quantifies them in a plurality of degrees (S10), detects the shape of the area and quantifies it (S11), and stores and transfers the resulting values (S12). Quantifying a shape involves, as mentioned previously, quantifying the length and smoothness of the contour line of a tongue with a view to detecting the size of teeth marks, and quantifying the size of a low-luminance area under an eye with a view to detecting a bag under the eye. By quantifying information on color and shape in this way, it is possible to make an objective comparison of the current condition with a past condition, and of one person's condition with another person's condition.

As described above, by imaging an imaging object with the imager 3 while it is lit with the first illuminator 2a, it is possible to include shadings in the taken image acquired by the imager 3 (see Figs. 4 to 6, 8, and 9). Based on those shadings, the detector 5 can accurately detect surface irregularities in a peripheral part of, or a part under, an organ, such as teeth marks on a tongue or a bag under an eye, and can also accurately detect the boundary of an organ, such as the contour (shape) of a tongue. Based on such accurate detection results, it is possible to make more accurate diagnoses. That is, a diagnosis can be made based on, in addition to information on the color and luster of an imaging object as conventionally available, also information on shapes, such as of teeth marks, a bag under an aye, and a tongue contour, and this helps improve the accuracy of diagnoses compared with conventional practice.

Under the illumination by the first illuminator 2a, the imager 3 images shadings formed in a peripheral part of an organ, and based on the shadings, the detector 5 detects surface irregularities in the peripheral part of the organ; thus, the surface irregularities can be detected accurately, and this helps improve the accuracy of a diagnosis based on the results of the detection. In particular, in a case where the organ is a tongue, and the surface irregularities are teeth marks on the tongue, it is possible to detect the teeth marks on the tongue accurately (see Fig. 8), and to improve the accuracy of a diagnosis.

Under the illumination by the first illuminator 2a, the imager 3 images shadings formed on a surface under an organ, and the detector 5 detects surface irregularities under the organ; thus, the surface irregularities can be detected accurately, and this helps improve the accuracy of a diagnosis based on the results of the detection. In particular, in a case where the organ is an eye, and the surface irregularities are a bag under (in a lower part of) the eye, the bag under the eye can be detected accurately (see Fig. 9), and this helps improve the accuracy of a diagnosis.

Under the illumination of the first illuminator 2a, the imager 3 images an organ (e.g., a tongue) and shadings formed in a peripheral part of it, and based on the image of the organ and the shadings taken by the imager 3, the detector 5 can, by extracting an area with luminance equal to or higher than a predetermined value, accurately detect the boundary (contour line) of the organ. In this way, it is possible to accurately recognize the shape of the organ, and to improve the accuracy of a diagnosis.

The imager 3 can perform both first imaging (imaging I) under the illumination by the first illuminator 2a and second imaging (imaging II) under the illumination by the second illuminator 2b, and can thereby acquire, through imaging II at a smaller illumination angle, an image of a region (e.g., a back region of the tongue) that is difficult to acquire through imaging I at a larger illumination angle. In this way, features (color, shape, etc.) in an image taken through imaging II can be detected by the detector 5, and this helps further improve the accuracy of a diagnosis.

### [Another Configuration of an Organ Imaging Device]

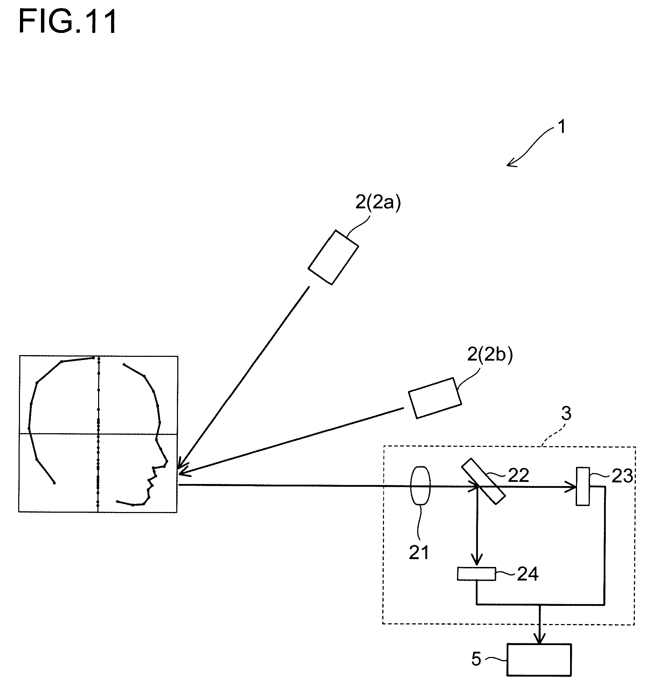

Fig. 11 is an explanatory diagram schematically showing another configuration of the organ imaging device 1. In the organ imaging device 1, the imager 3 can instead include an imaging lens 21, an infrared reflection mirror 22, a visible range sensor 23, and an infrared range sensor 24. In the illuminator 2, the first illuminator 2a can illuminate the imaging object with infrared or ultraviolet light, and the second illuminator 2b can illuminate the imaging object with visible light. In that case, the first and second illuminators 2a and 2b can be turned on simultaneously or one by one (at separate times). Although the following description deals with an example where the first illuminator 2a illuminates the imaging object with infrared light, it equally applies to a case where ultraviolet light is used for illumination.

In the imager 3, the imaging lens 21 focuses the illumination light emitted from the illuminator 2 and reflected from the imaging object (i.e., the reflected light) on the image sensing surface of the visible range sensor 23 or the infrared range sensor 24. The infrared reflection mirror 22 is a mirror (optical element) that splits the light incident through the imaging lens 21 according to wavelength. More specifically, the infrared reflection mirror 22 transmits, out of the light from the imaging object, visible light to direct it to the visible range sensor 23, and reflects infrared light to direct it to the infrared range sensor 24. The visible range sensor 23 is an image sensor (second image sensor) that receives visible light, and the infrared range sensor 24 is an image sensor (first image sensor) that receives infrared light.

In the above configuration, the infrared light emitted from the first illuminator 2a is reflected on the imaging object, is then transmitted through the imaging lens 21, is then reflected on the infrared reflection mirror 22, and then enters the infrared range sensor 24. The infrared range sensor 24 outputs, for each pixel, a signal representing the amount of infrared light received to the detector 5. On the other hand, the visible light emitted from the second illuminator 2b is reflected on the imaging object, is then transmitted through the imaging lens 21 and the infrared reflection mirror 22, and then enters the visible range sensor 23. The visible range sensor 23 outputs, for each pixel, a signal representing the amount of visible light received to the detector 5. Based on data containing the amounts of infrared and visible light received, the detector 5 extracts an area of the imaging object and thereby detects teeth marks or a bag under an eye in a manner similar to the one described previously.

By designing the first and second illuminators 2a and 2b to emit illumination light of different wavelengths, it is possible, as described above, to make the illumination light reflected from the imaging object (the reflected light) take separate optical paths according to wavelength. Thus, (to say nothing of a case where imaging is performed at separate times) even when imaging is performed simultaneously, an image taken under the illumination by the first illuminator 2a and an image taken under the illumination by the second illuminator 2b can be acquired quite separately. Even when infrared light is shone on the imaging object at an angle larger than 45 degrees, shadings can be formed in the taken image; thus, based on the shadings, the detector 5 can extract the area of a tongue, teeth marks on the tongue, or a bag under an eye. Infrared light is invisible to the human eye, and is thus not recognized by a user as an imaging object. The use of infrared light thus helps minimize the psychological burden on the user.

The imager 3 simultaneously performs first imaging (imaging I) under the illumination by the first illuminator 2a and second imaging (imaging II) under the illumination by the second illuminator 2b. This, as compared with performing imaging at separate times, helps improve the accuracy of a diagnosis on the same imaged part on the imaging object. That is, when imaging is performed at separate times, a shake on the part of the imaging object, or a shake (such as camera shake) on the part of the imager, produces a displacement between the two images taken at different illumination angles; this displacement then needs to be taken into consideration in a diagnosis, tending to degrade the accuracy of the diagnosis. Performing first and second imaging at the same time yields two taken images with no displacement with respect to the same part on the imaging object, and this eliminates the need to take a displacement into consideration in a diagnosis; thus it is possible, based on features detected from the two taken images, to improve the accuracy of the diagnosis with respect to the same part on the imaging object.

Owing to the imager 3 including the infrared reflection mirror 22 for separating optical paths of infrared and visible light, the infrared range sensor 24 which receives infrared light traveling along one of the separate optical paths, and the visible range sensor 23 which receives visible light traveling along the other of the separate optical paths, even when first and second imaging are performed simultaneously, the infrared and visible light can be separated by the infrared reflection mirror 22 so that the resulting taken images can reliably be acquired by the infrared range sensor 24 and the visible range sensor 23 respectively.

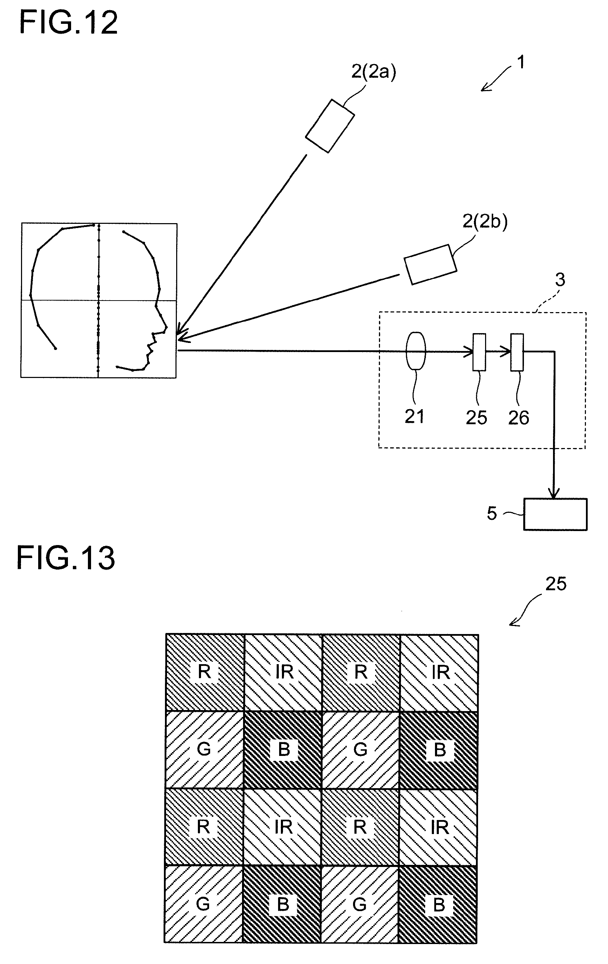

Fig. 12 is an explanatory diagram schematically showing yet another configuration of the organ imaging device 1. The imager 3 can instead include an imaging lens 21, a color filter 25, and an image sensor 26. The color filter 25 is arranged on the light incidence side of the image sensor 26, and transmits visible and infrared (or ultraviolet) light to direct it to the image sensor 26.

Fig. 13 schematically shows an outline of the structure of the color filter 25. The color filter 25 is composed of an array of filters (first filters) that transmit only visible light and IR filters (second filters) that transmit only infrared light. The filters that transmit only visible light comprise R filters, G filters, and B filters that transmit light of R, G, and B respectively. The image sensor 26 is composed of an array of pixels that receive light transmitted through the first filters of the color filter 25 and pixels that receive light transmitted through the second filters. Thus, the filters of the color filter 25 correspond one-to-one to the pixels of the image sensor 26. A configuration is also possible where the light that is transmitted through each filter of the color filter 25 is incident on a plurality of pixels (a group of pixels) of the image sensor 26 (each filter can correspond to a plurality of pixels of the image sensor 26).

In the color filter 25, instead of IR filters, UV filters that transmit ultraviolet light can be arranged. Whether to use as the color filter 25 one comprising IR filters or one comprising UV filters can be determined according to the type (infrared or ultraviolet light) of the illumination light of the first illuminator 2a.

In the above configuration, the infrared light emitted from the first illuminator 2a is reflected on the imaging object, and then passes through the imaging lens 21 to be incident on the color filter 25; the light transmitted through the second filters (IR filters) of the color filter 25 is incident on the pixels of the image sensor 26. These pixels of the image sensor 26 each output a signal representing the amount of infrared light received to the detector 5. On the other hand, the visible light emitted from the second illuminator 2b is reflected on the imaging object, and then passes through the imaging lens 21 to be incident on the color filter 25; the light transmitted through the first filters (R, G, and B filters) of the color filter 25 is incident on the pixels of the image sensor 26. These pixels of the image sensor 26 each output a signal representing the amount of R, G, or B light received to the detector 5. Based on data containing the amounts of infrared and visible light, the detector 5 extracts the area of the imaging object and thereby detects teeth marks or a bag under an eye in a similar manner as described previously.

By arranging the color filter 25 in the optical path of the illumination light (i.e., the reflected light) traveling from the imaging object to the image sensor 26 as described above, it is possible to acquire two taken images at different illumination angles simultaneously with a single image sensor 26. This eliminates the need for the infrared reflection mirror 13 and a sensor dedicated to the sensing of infrared light (the infrared range sensor 24) needed in the configuration shown in Fig. 11, and thus helps achieve size reduction and cost reduction in the device.

### [Modifications]

Although the above embodiment deals with a configuration where the illuminator 2 is composed of two illuminating mechanisms (the first and second illuminators 2a and 2b), instead a mechanism that allows an illuminating mechanism to be moved between different positions can be provided so that one illuminating mechanism will do. In this way, it is possible to reduce the number of illuminating mechanisms, and thereby to achieve size reduction and cost reduction in the device.

Although the above embodiment deals with an example where the image taken by the imager 3 is a color image having R, G, and B color information, it can instead be a monochrome image. This helps reduce the capacity of the storage for storing image data, and also helps increase the speed of processing for discriminating (detecting) features.

The organ imaging device according to the embodiment described above can be expressed as follows, and provides workings and effects as noted below.

An organ imaging device according to the embodiment includes an imager which images an imaging object including an organ of a living body to acquire an image, and a detector which detects a feature of the imaging object based on the image acquired by the imager. Here, the organ imaging device further comprises an illuminator which illuminates the imaging object at an angle larger than 45 degrees but equal to or smaller than 90 degrees relative to the imaging optical axis of the imager running through the imaging object. The imager images shadings formed by the imaging object under illumination by the illuminator. The detector detects a surface irregularity on the imaging object and the boundary of an organ included in the imaging object based on the shadings.

With the above configuration, while an imaging object is illuminated by the illuminator at an angle larger than 45 degrees but equal to or smaller than 90 degrees relative to the imaging optical axis, under such illumination, the shadings formed by the imaging object are imaged by the imager. These shadings include shadings resulting from an irregularity on the imaging object (e.g., in a peripheral part of, or in a region under, the organ) and the shadow of the imaging object (e.g., the organ) itself. The shadings permit the irregularity on the imaging object and the boundary of the organ to appear clearly in the taken image. Thus, by letting the detector detect the irregularity on the imaging object and the boundary of the organ included in the imaging object based on the shadings, it is possible, even when the surface irregularity on the imaging object are small, to detect them accurately, and to detect the boundary of the organ accurately. By making a diagnosis based on the results of the detection, it is possible to improve the accuracy of the diagnosis.

Preferably, the shadings are formed in a peripheral part of the organ, and the detector detects a surface irregularity in the peripheral part of the organ. The detector can then accurately detect the surface irregularity in the peripheral part of the organ based on the shading, and this helps improve the accuracy of a diagnosis based on the results of the detection.

Preferably, the organ is a tongue, and the surface irregularity is a tooth mark on the tongue. The detector can then accurately detect the tooth mark on the tongue, and this helps improve the accuracy of a diagnosis based on the tooth mark.

Preferably, the shadings are formed on a surface under the organ, and the detector detects a surface irregularity under the organ. The detector can then accurately detect the surface irregularity under the organ based on the shading, and this helps improve the accuracy of a diagnosis based on the results of the detection.

Preferably, the organ is an eye, and the surface irregularity is a bag under the eye. The detector can then accurately detect the bag under the eye, and this helps improve the accuracy of a diagnosis based on the bag.

Preferably, the imager images the organ and shadings formed in a peripheral part of the organ under illumination by the illuminator, and the detector extracts an area with luminance equal to or higher than a predetermined value from the image acquired by the imager. It is then possible to accurately detect the boundary between the organ and its surroundings; it is thus possible to accurately recognize the shape of the organ and improve the accuracy of a diagnosis.

Preferably, the illuminator illuminates the imaging object with infrared or ultraviolet light. Infrared or ultraviolet light is invisible to the human eye, and is not recognized as illumination by a user as an imaging object. This allows prompt imaging with the user in a relaxed state.

Preferably, the organ imaging device comprises the illuminator as a first illuminator and further comprises a second illuminator which illuminates the imaging object at an angle equal to or smaller than 45 degrees relative to the imaging optical axis. Here, the imager performs first imaging under illumination by the first illuminator and second imaging under illumination by the second illuminator.

By performing, in addition to first imaging under the illumination by the first illuminator from a large angle, second imaging under the illumination by the second illuminator from a small angle, it is possible to acquire, through imaging (second imaging) under illumination from a small angle, an image of a region (e.g., a back region of a tongue) which is difficult to acquire through solely imaging (first imaging) under illumination from a large angle. It is then possible, based on features (color, shape, etc.) in the image taken through second imaging, to further improve the accuracy of a diagnosis.

Preferably, the imager performs the first and second imaging simultaneously. By performing first and second imaging simultaneously, it is possible to eliminate the influence, which tends to occur when imaging is performed at separate times, of a shake on the part of the imaging object or a shake (such as camera shake) on the part of the imager. It is then possible, based on two images taken at different illumination angles, to improve the accuracy of a diagnosis with respect to the same part on the imaging object.

Preferably, the first illuminator illuminates the imaging object with infrared or ultraviolet light, and the second illuminator illuminates the imaging object with visible light.

By designing the first and second illuminators to emit illumination light of different wavelengths, it is possible to make the illumination light reflected from the imaging object (the reflected light) take separate optical paths according to wavelength, or to split the light incident on different pixels in the imager according to wavelength. It is thus possible to acquire quite separately an image taken under the illumination by the first illuminator and an image taken under the illumination by the second illuminator even when those images are taken simultaneously. Even when infrared or ultraviolet light is shone on the imaging object at an angle larger than 45 degrees, shadings can be formed in the taken image; thus, based on the shadings, it is possible to detect a surface irregularity. Moreover, since infrared or ultraviolet light is invisible to the human eye, imaging can be performed without a user as an imaging object recognizing the illumination light from a large angle.

Preferably, the imager includes an optical element which separates from each other the optical path of the infrared or ultraviolet light reflected from the imaging object after illuminating the imaging object and the optical path of the visible light reflected from the imaging object after illuminating the imaging object, a first image sensor which receives the infrared or ultraviolet light after optical path separation by the optical element; and a second image sensor which receives the visible light after optical path separation by the optical element.

Then, even when imaging under the illumination by the first illuminator and imaging under the illumination by the second illuminator are performed simultaneously, the optical path of the reflected light (infrared or ultraviolet light) originating from the illumination by the first illuminator and the optical path of the reflected light (visible light) originating from the illumination by the second illuminator can be separated from each other by the optical element, and thus images taken under different illumination can be reliably acquired by the first and second image sensors respectively.

Preferably, the imager includes a color filter having an array of first filters which transmit only infrared or ultraviolet light and second filters which transmit only visible light, and an image sensor having an array of pixels which receive light transmitted through the first filters and pixels which receive light transmitted through the second filters.

It is then possible to acquire two images taken at different illumination angles through the color filter at the image sensor. Thus, compared with a configuration where the illumination light traveling from the imaging object to the image sensor (i.e., the reflected light) is made to take separate paths according to wavelength by an optical element, or a configuration where separate image sensors are provided for the separate optical paths respectively, helps simplify the configuration of the device, and helps achieve size reduction and cost reduction in the device.

### Industrial Applicability

The present invention finds applications in devices that image an organ of a living body and a peripheral part of the organ to detect its features.

### List of Reference Signs

- 1: organ imaging device
- 2: illuminator
- 2a: first illuminator
- 2b: second illuminator
- 3: imager
- 5: detector
- 22: infrared reflection mirror (optical element)
- 23: visible range sensor (second image sensor)
- 24: infrared range sensor (first image sensor)
- 25: color filter
- 26: image sensor

## Claims

1. An organ imaging device comprising:
an imager which images an imaging object including an organ of a living body to acquire an image; and
a detector which detects a feature of the imaging object based on the image acquired by the imager,
wherein
the organ imaging device further comprises an illuminator which illuminates the imaging object at an angle larger than 45 degrees but equal to or smaller than 90 degrees relative to an imaging optical axis of the imager running through the imaging object,
the imager images shadings formed by the imaging object under illumination by the illuminator, and
the detector detects a surface irregularity on the imaging object and a boundary of an organ included in the imaging object based on the shadings.

2. The organ imaging device according to claim 1, wherein
the shadings are formed in a peripheral part of the organ, and
the detector detects a surface irregularity in the peripheral part of the organ.

3. The organ imaging device according to claim 2, wherein
the organ is a tongue, and
the surface irregularity is a tooth mark on the tongue.

4. The organ imaging device according to claim 1, wherein
the shadings are formed on a surface under the organ, and
the detector detects a surface irregularity under or the organ.

5. The organ imaging device according to claim 4, wherein
the organ is an eye, and
the surface irregularity is a bag under the eye.

6. The organ imaging device according to claim 1, wherein
the imager images the organ and shadings formed in a peripheral part of the organ under illumination by the illuminator, and
the detector extracts an area with luminance equal to or higher than a predetermined value from the image acquired by the imager.

7. The organ imaging device according to any one of claims 1 to 6, wherein the illuminator illuminates the imaging object with infrared or ultraviolet light.

8. The organ imaging device according to any one of claims 1 to 6, wherein
the organ imaging device comprises the illuminator as a first illuminator and further comprises a second illuminator which illuminates the imaging object at an angle equal to or smaller than 45 degrees relative to the imaging optical axis, and
the imager performs first imaging under illumination by the first illuminator and second imaging under illumination by the second illuminator.

9. The organ imaging device according to claim 8, wherein the imager performs the first and second imaging simultaneously.

10. The organ imaging device according to claim 9, wherein
the first illuminator illuminates the imaging object with infrared or ultraviolet light, and
the second illuminator illuminates the imaging object with visible light.

11. The organ imaging device according to claim 10, wherein the imager includes:
an optical element which separates from each other an optical path of the infrared or ultraviolet light reflected from the imaging object after illuminating the imaging object and an optical path of the visible light reflected from the imaging object after illuminating the imaging object;
a first image sensor which receives the infrared or ultraviolet light after optical path separation by the optical element; and
a second image sensor which receives the visible light after optical path separation by the optical element.

12. The organ imaging device according to claim 10, wherein
the imager includes:
a color filter having an array of first filters which transmit only infrared or ultraviolet light and second filters which transmit only visible light; and
an image sensor having an array of pixels which receive light transmitted through the first filters and pixels which receive light transmitted through the second filters.
